# EUROPEAN PATENT APPLICATION

(11) **EP 1 669 355 A1**
(43) Date of publication of application: **14.06.2006**
(21) Application number: 04028614.8
(22) Date of filing: 02.12.2004
(51) Int. Cl.: C07D 475/08, C07H 15/18

(54) **Aminotetrahydropteridines and processes for manufacture thereof**

(71) Applicant: Vasopharm Biotech GmbH, 97076 Würzburg (DE)
(72) Inventor: Noe, Christian, 1180 Wien (AT)
(74) Representative: Jones Day

(57) **Abstract**

The present invention relates to aminotetrahydropteridines and processes for the manufacture thereof. In particular, the present invention relates to a process for the manufacture of aminotetrahydrobiopterine. Said compound is an inhibitor of NO-synthase and may therefor be a valuable agent in medical applications. The manufacturing process starts from D-ribose, which is transformed in several reaction steps into an open chain osone structure with protected hydroxyl groups. Preferred protective groups are ether forming groups, in particular benzyl groups. Subsequent reaction with tetraaminopyrimidine yields hydroxyl-protected aminopteridines, which, after reduction to aminotetrahydropteridines and cleavage of the protective groups, yield the target compound. The invention also relates to said intermediates and a process for the manufacture thereof. Another subject-matter of the present invention is the use of D-ribose in said manufacturing processes.

## Description

The present invention relates to aminotetrahydropteridines and processes for the manufacture thereof. In particular, the present invention relates to a process for the manufacture of aminotetrahydrobiopterine. Said compound is an inhibitor of NO-synthase and may therefor be a valuable agent in medical applications. The manufacturing process starts from D-ribose, which is transformed in several reaction steps into an open chain osone structure with protected hydroxyl groups. Preferred protective groups are ether forming groups, in particular benzyl groups. Subsequent reaction with tetraaminopyrimidine yields hydroxyl-protected aminopteridines, which, after reduction to aminotetrahydropteridines and cleavage of the protective groups, yield the target compound. The invention also relates to said intermediates and a process for the manufacture thereof. Another subject-matter of the present invention is the use of D-ribose in said manufacturing processes.

(1'R,2'S)-1-[(6R*S*)-2,4-diamino-1,5,6,7-tetrahydro-6-pteridinyl]-1,2-propanediol (aminotetrahydrobiopterine) of the formula III 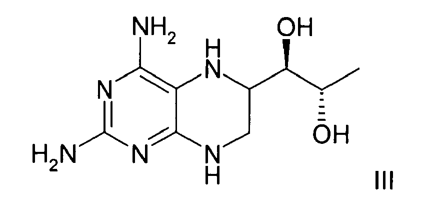 is a known compound. It antagonizes the effect of NO-synthase via a specific mechanism differing from that of other NO-synthase inhibitors. Therefore, said compound is a very promising candidate to treat diseases such as traumatic brain injuries or stroke. The synthesis presently applied to manufacture aminotetrahydrobiopterine starts from the compound biopterine introducing an amino group via several reaction steps (T. Hanaya, K. Torigoe, K. Soranaka, H. Yamamoto, Y. Qizheng, W. Pfleiderer, Pteridines 6 (1995) 1-7). Biopterine itself is a compound with limited availability and presently very high market prices, which poses a serious obstacle against the production of the compound in quantities required for clinical development and therapeutic practice.

It was an object of the invention to provide easily available starting materials and intermediates for the manufacture of the compound of the formula III as well as appropriate processes for the manufacture thereof.

This object could be achieved by means of intermediates manufactured by means ofD-ribose as the starting material.

One aspect of the invention relates to a compound of the general formula VIII 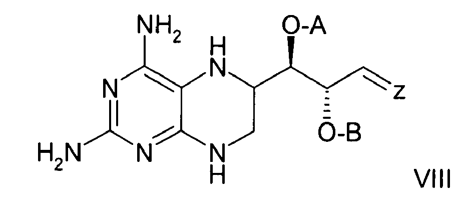 wherein A and B are independently from each other -H or protective groups for hydroxyl groups and =Z is an acyclic or cyclic dithioacetal moiety or =Z means - H, -H, with the proviso that the compound of the formula III is excluded.
The term "protective group" is a commonly used term in the field of organic chemistry. It stands for a collective name for organic radicals, which are able to temporarily protect active centres of a molecule against the attack of reagents. The term "protective groups for hydroxyl groups" means all protective groups A and B, which are suitable for protecting hydroxyl groups.

Preferably, such groups are selected from the group comprising the following groups: methyl, methoxymethyl, tetrahydropyran-2-yl, t-butyl, allyl, benzyl, t-butyldimethylsilyl, t-butyldiphenylsilyl, acetyl, pivaloyl and benzoyl.

The afore-mentioned groups are most frequently used as protective groups for hydroxyl groups.

Among those groups are such preferred, which form an ether with the hydroxyl groups to be protected.

Preferably, such ether forming groups are methyl, methoxymethyl, tetrahydropyran-2-yl, t-butyl, allyl, benzyl.

In particular, the protective groups A and B are benzyl groups .

The term "benzyl" means unsubstituted or substituted benzyl. More specifically, the term "benzyl" has the meaning of arylmethylen moieties -CH₂-Ar-Su, wherein said moieties can be the same or can be different from each other, and Ar has the meaning of a benzene ring, Su is from one to five substituents at the benzene ring and stands for branched or unbranched alkyl, branched or unbranched alkoxy, substituted or unsubstituted amino, halogen, nitro, cyano or trifluormethyl.

Preferably, Su is selected from the group consisting of unbranched or branched (C₁ to C₄)-alkyl or unbranched or branched (C₁ to C₄)-alkoxy, NH2, (unbranched or branched ( C₁ to C₄)- alkyl))NH, or (unbranched or branched (C₁ to C₄)-alkyl))₂N, chlorine, bromine or fluorine, nitro, cyano or trifluormethyl.

The term cyclic or acyclic "dithioacetal moiety =Z" preferably stands for (unbranched or branched alky)-S-, (unbranched or branched alky)-S-; Su-Ar-S-, Su-Ar-S-, wherein Su and Ar have the meaning as defined above; Su-Ar-CH₂-S-, Su-Ar-CH₂-S-, wherein Su and Ar have the meaning as defined above; or -S-(unbranched or branched alkylene)-S-.

Preferably, the dithioacetal moiety =Z consists of -S-(C₁ to C₄)-alkyl, -S-(C₁ to C₄)-alkyl or -SC₆H₅, -SC₆H₅.

In particular, =Z represents a cyclic dithiocetal moiety, preferably with a chain length of from 2 to 3 carbon atoms between the sulfur atoms (-S-CH₂-CH₂-S- or - S-CH₂-CH₂-CH₂-S- or -S-CH₂-CH(CH₃)-S-).

The compound of the general formula VIII may be used as an intermediate in the manufacturing process of the target compound of the formula III.

Another aspect of the invention relates to a compound of the general formula II, 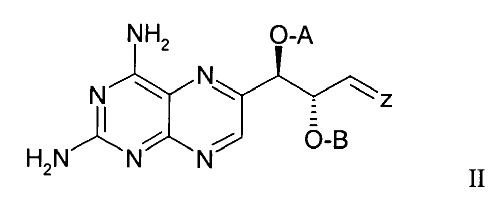 wherein A, B and =Z have the meaning as defined above.

The compound of the general formula II may be used as an intermediate in the manufacturing process of the compound of the general formula VIII or for the target compound of the formula III.

Another aspect of the invention relates to a compound of the general formula I 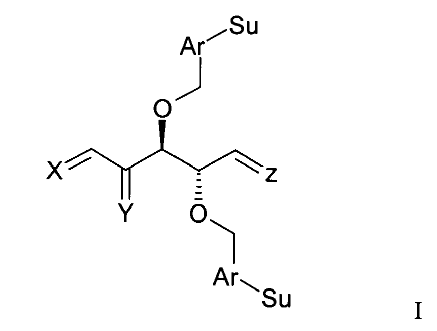 wherein -CH₂-Ar-Su has the meaning of a protective group A and B as defined above, =Z has the meaning as defined above, and =X has the meaning of =O, =NOR₁, =NNHR₁, with R₁ meaning H, unbranched or branched alkyl, unsubstituted or substituted phenyl, in which =Y is =O or has the same meaning as =X and has in addition the meaning of -H, -OH, in which =X may be either equal or unequal to =Y.

If =Y stands for -H,-OH, then said secondary hydroxyl group has the configuration of the corresponding hydroxyl group of D-ribose, the compound of the general formula I is based on.

If R₁ is unbranched or branched alkyl, then, preferably, the alkyl radical is (C₁ to C₄)-alkyl, or if R₁ is substituted phenyl, then, preferably, said phenyl radical is substituted with one or more substituents Su as defined above.

The compound of the general formula I may be used as an intermediate in the manufacturing process of the compounds of the general formulas II and VIII or for the target compound of the formula III.

The starting materials for the reaction sequence is D-ribose which is a commercially available compound (e. g. Sigma Aldrich Fine Chemicals).

In said reaction sequence, said sugar is transformed in several steps into an open chain osone structure of the general formula I. Herein, compounds are shown, wherein the hydroxyl groups are preferably protected by benzyl groups -CH₂-Ar-Su as defined above. A subsequent reaction with tetraaminopyrimidine yields the protected aminopteridines of the general formula II, which, after reduction to aminotetrahydropteridines of the general formula VIII and cleavage of the protective groups, yield the target compound of the formula III.

Exemplarily, a suitable reaction sequence is given below.

In the first step, a dithioacetal derivative of the general formula IV 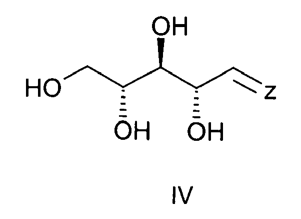 is prepared, wherein =Z has the meaning of said dithioacetal moiety as defined above, using as the starting material D-ribose and an appropriate reagent, which is able to form a thioacetal or thioketal.

As preferred thioketal forming agents, ethanedithiol or 1,3-propanedithiol are employed.

Thioacetalisation of D-ribose readily proceeds under strong acid catalysis. Such a reaction is well known in the art. For example, D-ribose can be reacted with ethanedithiol in solution using hydrochloric acid as the catalyst (H. Zinner; Chem. Ber., 83, 1950; 275,276).

In the following step, selective ketal formation with the hydroxyl groups of the positions 4 and 5 of the D-ribose derivative of formula IV, with a carbonyl compound suited as a ketal type protective group is carried out to yield a compound of the general formula V 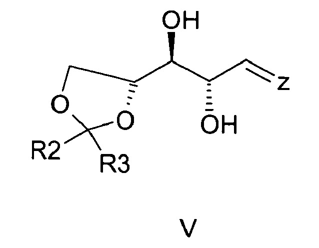 wherein =Z has the same meaning as defined for the compound of the general formula IV and wherein R₂ and R₃ are independently from each other branched or unbranched alkyl, substituted or unsubstituted aryl and H.

Preferably, branched or unbranched alkyl is (C₁ to C₄)-alkyl.

Preferably, unsubstituted or substituted aryl is the phenyl radical or a phenyl radical bearing one or more substituents Su as defined above.

In particular, benzaldehyde or acetone may be used as acetal or ketal forming reagents.

The formation of acetales or ketales from aldehydes and ketones with alcohols is a known reaction. Typically, the above reaction proceeds under the influence of strong acids such as sulfuric acid in solution (O. Kölln, H. Redlich; Synthesis, 1995, 1381).

Compound IV has two hydroxyl groups, which are left free in the above two reaction steps. Said groups are foreseen for subsequent protection, preferably by benzyl ether type protection.

However, prior to the protection reaction, the dithioacetal moiety may be removed by desulphurisation. Such a reaction type is already known and can be carried out by known methods. Preferably, Urushibara Raney-Nickel, which can be prepared according to known methods from precipitated nickel and zinc dust in water, may be used as desulphurisation reagent. Normally, the reaction is carried out in a basic medium, e. g. using sodium carbonate, and in an organic solvent, e. g. an alcohol. In common, the reaction already proceeds at room temperature. The desulphurised compound can be isolated by removing the solvent. It may be used as the crude in the further processing.

After desulphurisation, a compound V is obtained, wherein =Z is -H, -H. However, it is possible to remove said dithioacetal group in the reaction sequence in a later point of time, if required or desired.

The protection of alcohols in form of a benzyl ether is already known and can be carried out by known methods. Benzyl groups may be introduced by deprotonation of hydroxyl groups in aprotic solvents followed by alkylation with an arylalkylhalide according to common processes.

Therefore, benzyl groups -CH₂-Ar-Su may be introduced by deprotonation of the hydroxyl groups of compounds V in aprotic solvents like DMF, DMSO, diglyme, or THF followed by alkylation with an arylalkylhalide like benzylbromide or benzylchloride to yield a compound of the general formula VI 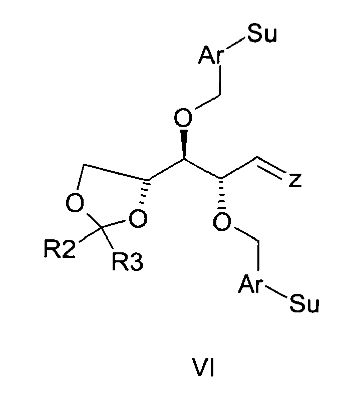 wherein R₂, R₃ have the same meaning as given for the compound of the formula V and -CH₂-Ar-Su and =Z have the meaning as given for the compound of the general formula I. In case that =Z stands for -H, -H meaning assigning the reaction step of reductive cleavage of the dithioacetal group prior to the benzylation reaction as described before.

In a subsequent reaction step, cleavage of the ketal group results in protected sugar derivatives of the general formula VII 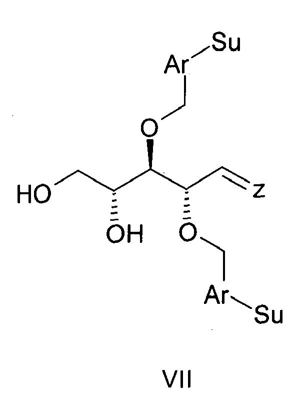 wherein Ar and Su have the same meaning as given for a compound of the general formula I, and in which =Z has the meaning of a dithioacetal moiety as defined above, or, in case of a preceding desulphurisation reaction, has the meaning of - H,-H.

The next reaction step comprises an activation step of the hydroxyl groups in the compound of the general formula VII in order to allow a later subsequent ring closure reaction to the pteridine system.

The most appropriate activation is achieved by oxidation. Herein, a compound of the general formula VII yields the L-ribose osone of the general formula I, wherein =X and =Y mean =O. Said osone has the structure of a keto aldehyde.

Said osone may be obtained in a one step reaction by an appropriate oxidation reaction, preferably an oxidation of the known Swern reaction type. Said oxidation type allows the selective oxidation of primary and secondary hydroxyl groups without the formation of carboxylic acid. The reaction is carried out with a mixture of sulfones or sulfoxides, e.g. DMSO, with oxalyl chloride in the presence of a base. Typically, the reaction proceeds very smoothly in a solvent like chlorinated hydrocarbons, e.g. dichloromethane, in a temperature range of from -100 °C to 50 °C, preferably -70 °C to 30 °C. The resulting product may be isolated from the reaction mixture by distilling off the solvent. The crude product may be purified by recrystallisation or may be processed as the crude product.

Though the osone may be processed directly with tetraaminopyrimidine to the aminopteridine system, however, the compound of the general formula II may not be the main product and may only be isolated in a minor amount. Therefore, preferably, said osone is modified prior to said ring closure reaction.

The osone can be modified and transformed according to common procedures by reaction with substituted or unsubstituted hydroxylamines or hydrazines to monoximes, dioximes, their mixtures or hydrazones of the general formula I, wherein =X has the meaning =NOR₁, =NNHR₁, with R₁ meaning H, unbranched or branched alkyl, unsubstituted or substituted phenyl, in which =Y is O or has the same meaning as =X with the definitions given above.

For example, the osone can be reacted with hydroxylamine hydrochloride or phenyhydrazine in an organic solvent like methanol or ethanol under the influence of a base like hydrochloric acid or p-toluenesulphonic acid to the respective products. Said products may be isolated by distilling off the solvent and purifying the residue by common chromatographic methods, e. g. by flash-chromatography.

However, it is also possible first oxidising said primary hydroxyl group of the compound of the general formula VII. Afterwards, the resulting aldehyde group can be derivatized in the above-mentioned manner. Such a compound may be reacted with tetraaminopyrimidine, and, upon addition of an oxidising reagent, such as iodine, the secondary hydroxyl group may be oxidised and ring closure may occur. Oxidation and ring closure are then carried out in the manner of an in situ-reaction.

Still another aspect of the invention relates to a process for the manufacture of a compound of the general formula I, wherein Ar and Su have the meaning as defined above, comprising steps (i) to (vi):
(i) transformation of D-ribose into a dithioacetal of the general formula IV, 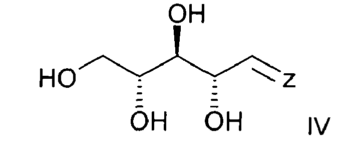 wherein =Z has the meaning of said dithioacetal moiety as defined above,
(ii) ketal formation of compound IV to yield a compound of the general formula V, 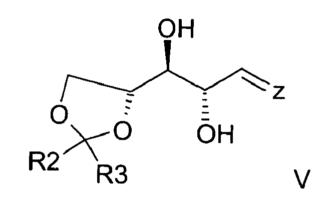 wherein R₂ and R₃ are independently from each other branched or unbranched alkyl, substituted or unsubstituted aryl or H,
(iii) benzyl ether type protection of the OH-groups of compound V to yield a compound of the general formula VI, 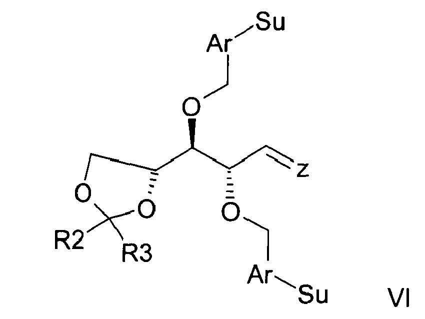 wherein -CH₂-Ar-Su have the same meaning as defined for a compound of the general formula I, and wherein =Z has the meaning of said dithioacetal moiety or stands for -H, -H, in the latter meaning assigning a reaction step of a reductive cleavage of said dithioacetal group prior to the introduction of -CH₂-Ar-Su,
(iv) cleavage of the ketal group in compound VI to yield protected sugar derivatives of the general formula VII 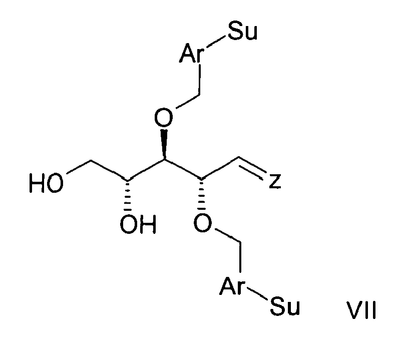
(v) subsequent step of an oxidation reaction to yield a compound of the general formula I, wherein =X and =Y are O,
(vi) and optionally modification of the compound obtained in step (v) to form a monoxime, dioxime or hydrazone to yield a compound of the general formula I, wherein =X has the meaning =NOR₁, =NNHR₁, with R₁ meaning H, unbranched or branched alkyl, unsubstituted or substituted phenyl,
in which =Y is =O or has the same meaning as =X, as defined above.

The compounds of the general formula I exhibit an open chain structure containing 5 consecutive carbon atoms. Upon ring fusion with tetraaminopyrimidine, aminobiopterines of the general formula II are formed.

Tetraaminopyrimidine is a known substance and is commercially available (e.g. from Sigma Aldrich Fine Chemicals). It may be applied in the form of a salt, e.g. as the salt with two equivalents of hydrochloric acid or with sulphuric acid.

Preferably, ring closure is carried out in an condensation reaction under acidic reaction conditions using the monoxime, dioxime, their mixtures or hydrazone of compounds of formula I. With said compounds, in general a high position selectivity of the side chain in the ring closure reaction is achieved. It should be emphasised that an advantageous ring formation occurs with perfect configuration of the chiral centres of the side chain, which is required for the manufacture of a compound of the formula III.

Therefor, the ring closure reaction is preferably carried out with the modified osone and not with the osone itself. Generally, said reaction is carried out in a solvent like an alcohol, like methanol or ethanol, which contains hydrochloric acid, employing temperatures preferably in the range of from 50 to 120 °C, preferably reflux temperature.

The resulting compounds of the general formula II tend to crystallise well from the reaction mixture or are obtained by distilling the solvent off. The crude compounds can be purified by recrystallization.

Therefore, another aspect of the invention relates to a process for the manufacture of a compound of the general formula II as defined above, comprising step (i)
(i) reaction of tetraaminopyrimidine with a compound of the general formula I as defined above or with a compound of the general formula I manufactured according to the above process.

The aminotetrahydropteridines of the general formula VIII 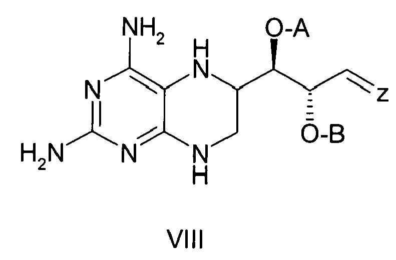 as defined above can be obtained from compounds of the general formula II by a hydrogenation reaction.

Thus, another aspect of the invention relates to a process for the manufacture of a compound of the general formula VIII, wherein A, B and Z have the meaning as defined above, comprising steps (i) and (ii):
- (i): reaction of tetraaminopyrimidine with a compound of the general formula I as defined above or with a compound of the general formula I manufactured according to the above process,
- (ii): hydrogenation of the compound with the general formula II obtained in step (i).

The hydrogenation reaction may be carried out using hydrogen and a catalyst. Since in the catalytic hydrogenation using a platinum catalyst the reduction of the pteridine ring precedes the cleavage of the protective group the option is given, either to perform both steps in one reaction or to cleave the benzyl protective group in a separate step.

Hydrogenation of a compound of formula II, bearing a benzyl type protective group and with =Z in the meaning of -H, -H, using 4 molar equivalents of hydrogen results directly in the compound aminotetrahydrobiopterine (formula III) by reduction of the double bond and cleavage of protective groups in one step.

Preferably the hydrogenation reaction is a catalytic hydrogenation method under acidic conditions, wherein the compound of the general formula III is obtained.

For example, according to common methods, a platinum oxide catalyst can be employed. The reaction can be carried out in an alcohol, e. g. ethanol, using said catalyst, which is saturated with hydrogen. The reaction may be carried out at normal pressure and at room temperature. After the uptake of the calculated and desired hydrogen amount, the catalyst may be removed by filtration. Afterwards, the solvent is partially distilled off, and the desired compound may be obtained by precipitation and subsequent filtration.

Another aspect of the invention relates to a process for the manufacture of the compound of the formula III comprising the afore-mentioned process for the manufacture of a compound of the formula VIII, wherein step (ii) is carried out by a catalytic hydrogenation method under acidic conditions, preferably using a platinum catalyst.

The reduction of the pteridine ring to the tetrahydro derivatives may also be carried out by the use of other substances, which can supply hydrogen. among those substances, borane reagents may be mentioned, such as sodium boranate. Under such conditions, in general, the benzyl protective groups remain uncleaved.

The stability of benzyl ethers towards a variety of reaction conditions renders them particularly useful in the total synthesis of a multifunctional pteridine derivative like aminotetrahydrobiopterine.

According to known methods, depending on their substitution pattern, however, benzyl ethers may be cleaved from the hydroxyl group by different methods, e.g. hydrogenation, oxidation, acid hydrolysis or irradiation. Said methods are well known in the art of protective group chemistry.

The option of benzyl cleavage under non acidic conditions also provides the option to cleave the protective group before reduction of the pteridine ring, such opening a further option to reaction performance by yielding compounds of general formula II with A and B having the meaning of H.

In a subsequent step, in said protected derivatives, the benzyl protective group can be removed under acidic conditions, or under non-acidic conditions like oxidation.

In case that the benzyl group carries a nitro group, cleavage of said ether group can also be carried out by means of a photochemical reaction, i.e. by irradiation.

Another aspect of the invention relates to a process for the manufacture of the compound of the formula III, comprising the process of the manufacture of a compound of the general formula VIII, further comprising step (iii):
- (iii): cleavage of the protective groups by acid hydrolysis, oxidation or irradiation.

The tetrahydro derivatives of the general formula VIII or the target compound of the formula III can be isolated as salts, solvates or clathrates or mixtures thereof. Preferably, the compounds are isolated as hydrochlorides. They may be recrystallised to achieve further purification of the product.

As shown before, the target compound of the formula III and the intermediates of the general formulas I, II and VIII can be easily prepared from an easily available substance, i.e D-ribose.

Therefore, the invention also relates to the use of D-ribose for the manufacture of a compound of the general formula I or for a compound of the general formula II or for a compound of the general formula VIII or for a compound of the formula III or for the manufacture of a compound of the general formula I, II or VIII using the processes, which are described above.

Another aspect of the invention relates to a process for the manufacture of said compounds comprising the use of D-ribose.

As shown above, said oxidation of the terminal hydroxyl group in the compound of the general formula VII together with the reductive cleavage of the thioacetal group as described above formally defines the "switch" in configurational assignment to the L-sugar series starting from D-ribose. Thus, the compound of the general formula I can be regarded as a key intermediate in the synthesis of the target compound of the formula III.

Though the manufacturing process of the target compound of the formula III is shown in the above in detail via the intermediates of the general formula I, II and VIII, wherein the protective groups are a -CH₂-Ar-Su moiety, preferably benzyl, it is conceivable to carry out said reaction sequence with said other protective groups for hydroxyl groups. For example, it is conceivable to introduce a methyl group by using methylhalogenide or diazomethane, or a methoxymethyl group by using methoxymethylchloride, or the tetrahyropyrane-2-yl group by using 3,4-dihydro-2H-pyrane, or a t-butyl group by using iso-butene, or a silyl group by using an appropriate silyl chloride, or an acetyl group by using acetyl chloride or anhydride. Such methods are well known in the art. For a skilled person, it would be a matter of routine experiments to optimise the reaction conditions with respect to said protective groups, preferably with respect to reagents, which form protective groups of the ether type.

The following experiments describe the invention exemplarily.

### Examples

### Example 1: Dithioacetal formation

### Preparation of D-Ribose-1,2-ethandiyl-dithioacetal

### (Formula IV: =Z = -S-CH₂-CH₂-S-)

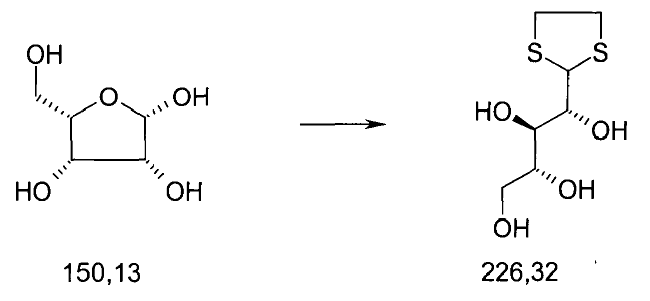

A solution of 100 g D-(-)-Ribose in 100 mL 6N HCl was cooled to 5 °C. 50 mL Ethanedithiol was added slowly so that the internal temperature did not exceed 10 °C. The resulting clear yellowish solution was stirred at room temperature for 16 hours whereupon a dense colourless suspension of the dithioacetal was obtained. The precipitate was filtered with suction. The residue (130 g) was dissolved in 400 mL of boiling 96 % ethanol, the solution was filtered while hot from a small amount of insoluble material and allowed to crystallise under cooling. The resulting colourless crystals were filtered, washed with diethyl ether, and dried. Yield: 99,2 g.

TLC (chloroform/methanol/water 85 : 20 : 2): (visualization of substance spots with phosphomolybdate-reagent). R_{f} (D-Ribose): 0,2 R_{f} (Product): 0,53.

### (Lit: H. Zinner; Chem. Ber., 83, 1950; 275,276)

### Example 2: Ketal formation

### Preparation of D-Ribose-4,5-O-(1-methylethyliden)-1,2-ethandiyl dithioacetal

### (Formula V: R₂ = R₃ = CH₃; =Z meaning -S-CH₂-CH₂-S-)

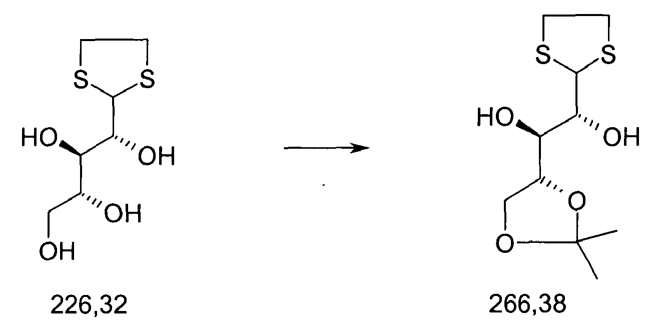

To a solution of 95 g dithioacetal in 1425 mL acetone, 50 µl conc. sulphuric acid were added at -5 °C. After stirring for 1,5 hours 1,5 mL 25 % (19,6 mmol) ammonium hydroxide solution were added, followed by 10 g potassium carbonate. The mixture was evaporated to dryness in vacuo. The residue was dissolved in 1000 mL dichloromethane, filtered from insoluble material. The solvent was evaporated. The resulting crude product (117 g of a pale yellow oil) was stirred with 130 mL diethyl ether for 12 hours at room temperature. The precipitate was filtered, washed with 50 mL diethyl ether and dried. Yield: 82 g colourless crystals.

TLC-chromatography (Eluent: chloroform/methanol/water 85 : 20 : 2); (visualization with phosphomolybdate-reagent) R_{f} (Dithioacetal): 0,5; R_{f} (Product): 0,83.

¹H-NMR (CDCl₃): δ = 4,95 (d, J = 5,2 Hz, 1 H), 4,39 - 4,30 (m, 1H), 4,11 - 3,92 (m, 2H), 1,44(s, 3H), 1,37 (s, 3 H); ¹³C-NMR (CDCl₃): δ = 109,17, 75,55, 75,48, 65,29, 55,78, 38,68, 38,24, 26,42, 25,20.

### (Lit: O. Kölln, H. Redlich; Synthesis, 1995, 1381)

### Example 3: Desulphurisation of dithioacetonide

### Preparation of 1,2-O-(1-methylethyliden)-5-desoxy-L-ribitol

### (Formula V: R₂ = R₃ = CH₃; =Z meaning -H, -H)

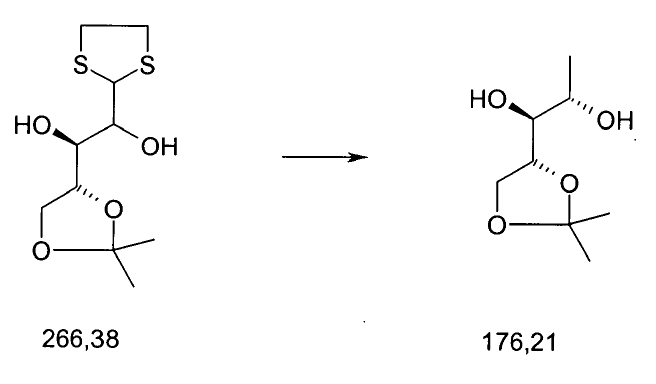

To a solution of 30 g dithioacetal in 350 mL ethanol, 26,25 g of sodium carbonate were added. Then a total of 205 g of a Urushibara Raney-Nickel suspension in water was added during 1,5 hours with efficient stirring. (Reaction control by TLC: dichloromethane/methanol 20 : 1) After complete reaction, the supernatant was decanted from the catalyst. The residue was stirred twice with 200 mL ethanol and the supernatant was decanted. The combined decanted ethanolic phases were filtered over a Celite-bed under nitrogen and evaporated in vacuo. Residual water was removed by azeotropic distillation with toluene. The residue was taken up in 120 mL ethanol, filtered, evaporated and dried. Yield: 18 g pale yellow oil.

TLC: (visualization with phosphomolybdate-reagent): R_{f} (Dithioacetonide): 0,45; R_{f} (Product): 0,3.

¹H-NMR (CDCl₃): δ = 4,02 - 3,88 (m 3 H), 3,58 3,53 (m, 1 H) 1,35 (s, 3H), 1,28 (s, 3H) 1,15 (d, J = 6,44 Hz, 3 H); ¹³C-NMR (CDCl₃): δ = 108,94, 76,07, 74,60, 68,55, 66,41, 26,55, 25,22, 17,42.

### Example 4: Benzylation reaction

### Preparation of 3,4-O-Dibenzyl-1,2-O-(1-methylethylidene)-5-desoxy-L-ribitol (Formula VI: R₂ = R₃ = CH₃; Ar = phenyl; Su meaning -H; =Z meaning -H, - H)

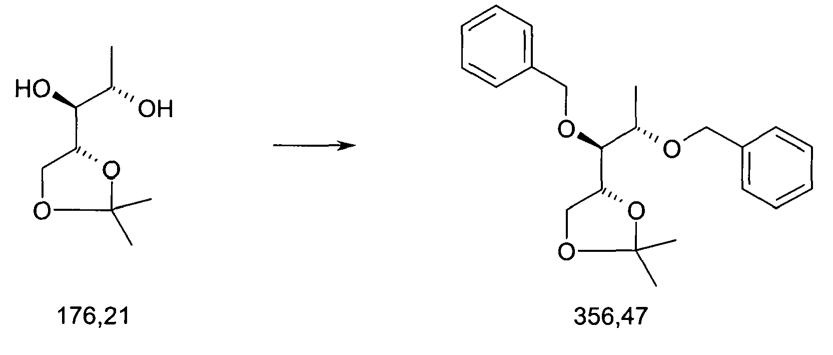

A solution of 18 g dihydroxyacetonide in 100 mL water free DMF was cooled to 0 - 5 °C under nitrogen atmosphere. 6,45 g Sodium hydride (95 %) were added in portions. After complete addition the reaction mixture was warmed to 40° C and stirred for 30 minutes. After cooling to 0 - 5 °C again, 34 mL benzylbromide were added slowly. After completion, the mixture was stirred at 60 °C for 1 hour, poured on 1200 mL water and extracted three times with 150 mL each of dichloromethane. The combined organic phases were washed with saturated sodium chloride solution, dried over sodium sulphate, filtered and evaporated to dryness. Yield: 36 g orange coloured oil, which might be used for the next step without further purification. TLC: Dichlormethan/Methanol 20 : 1; (visualization with phosphomolybdate-reagent); R_{f} (Educt): 0,2; R_{f} (Product): 0,6.

¹H-NMR (CDCl₃): δ = 7,36 - 7,2 (m, 10 H), 1,38 (s, 3H), 1,33 (s, 3H), 1,26 (d, J = 6,30 Hz); ¹³C-NMR (CDCl₃): δ = 138,67, 138,58, 128,37, 128,31, 128,28, 127,80, 127,75, 127,56, 127,49, 127,46, 108,85, 81,48, 75,65, 75,41, 26,48, 25,31, 18,44.

### Example 5: Deprotection of the acetonide

### Preparation of 3,4-0-Dibenzyl-5-desoxy-L-ribitol

### (Formula VII: R₂ = R₃ = CH₃; Ar = phenyl; Su meaning -H; =Z meaning -H, -H)

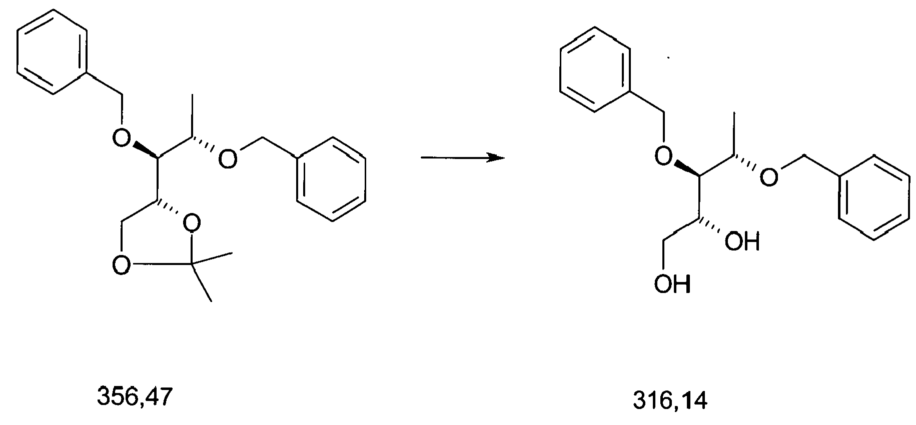

A solution of 36 g benzylation product in 400 mL 80 % acetic acid was stirred at 60 - 65 °C for one hour. Then the reaction mixture was cooled to 10 - 15 °C and a solution of 216 g sodium hydroxyde in 800 mL water were added slowly to neutralize acetic acid. Subsequently the mixture was rendered alkaline with saturated sodium bicarbonate solution. The aqueous solution was extracted with dichloromethane. Combined organic phases were dried over sodium sulphate, filtered and evaporated. The crude product was purified by flash chromatography over 500 g silica gel 60, first with petroleum ether/MTBE (methyl-t-butyl-ether) 4 : 1, then with petroleum ether/MTBE 2 : 1 as eluent. Yield: 14 g yellow oil.

TLC: Eluent: tert.-Butylmethylether/petroleum ether 1: 1; (visualization with phosphomolybdate-reagent); R_{f} (Educt): 0,7; R_{f} (Product): 0,1.

¹H-NMR (CDCl₃): δ = 7,23 (s, 10 H), 4,69 - 4,45 (m, 4H), 1,32 (d, J = 6,30 Hz, 3 H); ¹³C-NMR (CDCl₃): δ = 137,95, 137,86, 128,46, 127,99, 127,86, 1327,80, 127,71, 81,90, 76,51, 74,06, 72,47, 70,96, 15,86.

### Example 6: Oxidation reaction

### Preparation of 3,4-O-dibenzyl-5-desoxy-L-erythro-pentos-2-ulose (Osone)

### (Formula I: =X and =Y meaning =O; Ar meaning Phenyl; Su meaning -H; =Z meaning -H, -H)

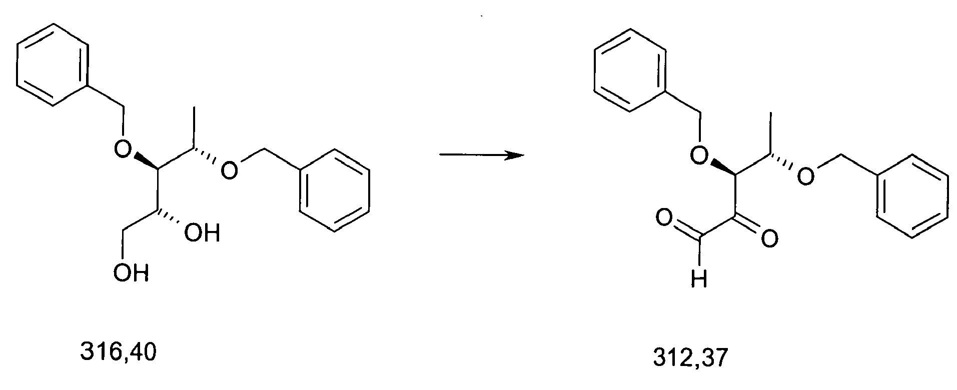

A solution of 6.12 g oxalyl chloride in 60 mL dry dichloromethane was cooled to -70 °C under argon atmosphere. A mixture of 4.06 g dry DMSO and 5 mL dry dichloromethane was added at an internal temperature below -65 °C. After addition the solution was stirred at -70 °C for 15 minutes. Then a solution of 7 g of 3,4-O-dibenzyl-5-desoxy-L-ribitol in 70 mL dry dichloromethane was added slowly, keeping the internal temperature below -65 °C. After an additional hour at -65 to -70 °C, then 33,7 mL triethylamine were added at a temperature below - 45° C. After stirring for further 20 minutes, the reaction was allowed to warm to room temperature, 150 mL dichloromethane and 200 mL water were added and phases are separated. The aqueous layer was further extracted with dichloromethane and the combined organic phases were washed with 2N acetic acid, 5% sodiumbicarbonate solution and saturated sodium chloride solution. The organic phase was dried, filtered and evaporated to yield 6,8 g orange oil, which might be used for transformation into other compounds of general formula I, such as monoxime, dioxime or phenylhydrazone without further purification.

### Example 7a - 7c: Modification of the osone of general formula I

### 7a: Monoxime formation:

### Preparation of 3,4-O-Dibenzyl-5-desoxy-L-erythro-pentos-2-ulose-1-oxime

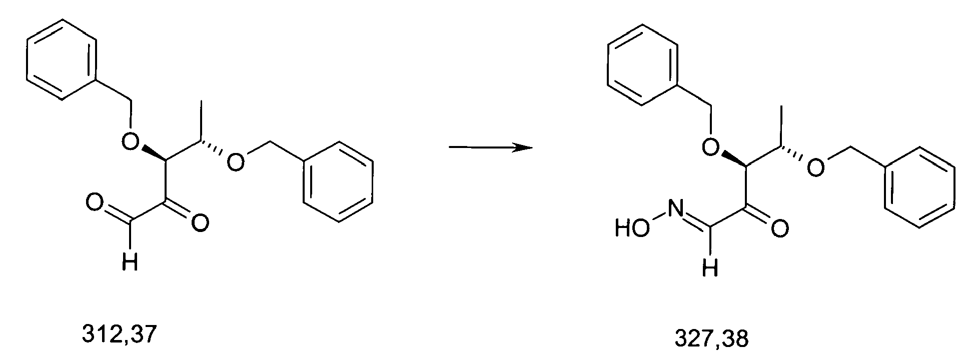

To a solution of 6,8 g crude ketoaldehyde in 50 mL methanol 1,17 g hydroxylamine hydrochloride and 1,38 g anhydrous sodium acetate were added in three equal portions at room temperature within one hour. The reaction mixture was diluted with MTBE and extracted with 0,5N HCl and 5 % sodium bicarbonate solutions. The combined organic phases were dried, filtered and the solvents were removed in vacuum.

Yield: 6,96 g orange oil. The crude product was purified by flash-chromatography (150 g silica gel, petroleum ether/MTBE 7 :1 and petroleum ether/MTBE 6 : 1). Yield: 2,66 g monoxime, yellow viscous oil and 0,52 g dioxime, yellow oil. TLC: dichloromethane/ethyl acetate 20 : 1; R_{f} (Starting material): ~0,85; R_{f} (Monoxime): 0,45; R_{f} (Dioxime): 0,2.

¹H-NMR (CDCl₃): δ = 7,63 (s, 1 H), 1,25 (d, J = 6,30 Hz, 3 H); ¹³C-NMR (CDCl₃): δ = 197,6, 148,30, 139,38, 137,62, 137,46, 137,03, 82,08, 75,81, 75,56, 71,23, 16,22.

### 7b: Dioxime formation

From 4 g of crude ketoaldehyde, 1,83 g hydroxylamine hydrochloride and 2 g anhydrous sodium acetate according to the procedure given in example 7a: Yield: 1,92 g dixome.

### 7c: Phenylhydrazone formation

To a solution of 0,6 g of crude ketoaldehyde in 6 ml methanol 0,2 g of phenylhydrazine were added at room temperature. Catalytic amounts of p-toluenesulphonic were added. After 3 hours methanol was distilled off and the residue subjected to flash chromatography as given in example 7a. Yield: 0,14 g of orange crystals.

### Example 8a- 8c: Ringclosure Reactions:

### 8a: Reaction of monoxime with 2,4,5,6-tetraaminopyrimidine dihydrochloride

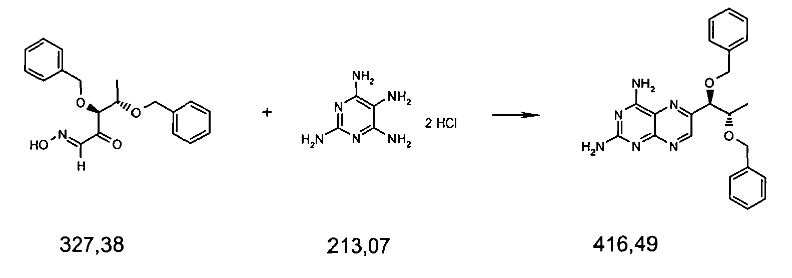

A solution of 1,26 g of conc. hydrochloric acid in 40,5 mL methanol was prepared. 0,62 g tetraaminopyrimidine dihydrochloride were suspended in 80 mL of methanol and heated to reflux. 27 mL of methanolic HCl solution were added via syringe while reflux was maintained. To the refluxing solution 0,96 g monoxime, dissolved in 25 mL methanol were added over a period of 40 minutes, followed by the addition of 13,5 mL methanolic HCl solution. The reaction mixture was refluxed for another two hours. The solvent was evaporated in vacuo, the residue was resolved in dichloromethane, the organic phase was extracted with 5% sodium bicarbonate solution, the aqueous phase was reextracted twice with dichloromethane. The combined organic phases were dried and evaporated to dryness. Yield: 0,97 g crude product as orange crystals. Upon trituration with MTBE and recrystallization from ethanol 0,36 g light yellow crystals were obtained. The solvents from triturating and recrystallization were combined and purified by flash chromatography to yield further 0,25 g light yellow crystals. TLC (ethyl acetate/ethanol 15 : 1): R_{f} (Monoxime): 0,9; R_{f} (Product): 0,5.

¹H-NMR (MeOD): δ = 8,66 (s, 1 H), 4,52 - 4,49 (m, 4 H) 1,30 (d, J = 6,30 Hz, 3 H); ¹³H-NMR (MeOD): δ = 165,71, 165,22, 156,99, 152,22, 150,55, 140,24, 139,91, 85,21, 73,97, 73,12, 17,23.

### 8b: Reaction of dioxime with 2,4,5,6-tetraaminopyrimidine dihydrochloride

From 0,31 g tetraaminopyrimidine dihydrochloride and 0,50 g dioxime according to procedure in example 8a with a reaction time of 20 hours. Yield after flash chromatography 0,19 g (recovery of 0,12 g of dioxime from the flash chromatography).

### 8c: Reaction of phenylhydrazone with 2,4,5,6-tetraaminopyrimidine dihydrochloride

From 0,1 g tetraaminopyrimidine dihydrochloride and 0,14 g phenylhydrazone according to procedure for example 8b. Yield after flash chromatography 0,04 g light yellow crystals.

### Example 9: Hydrogenation

### Preparation of (1'R,2'S)-1-[(6R*S*)-2,4-diamino-1,5,6,7-tetrahydro-6-pteridinyl]-1,2-propanediol

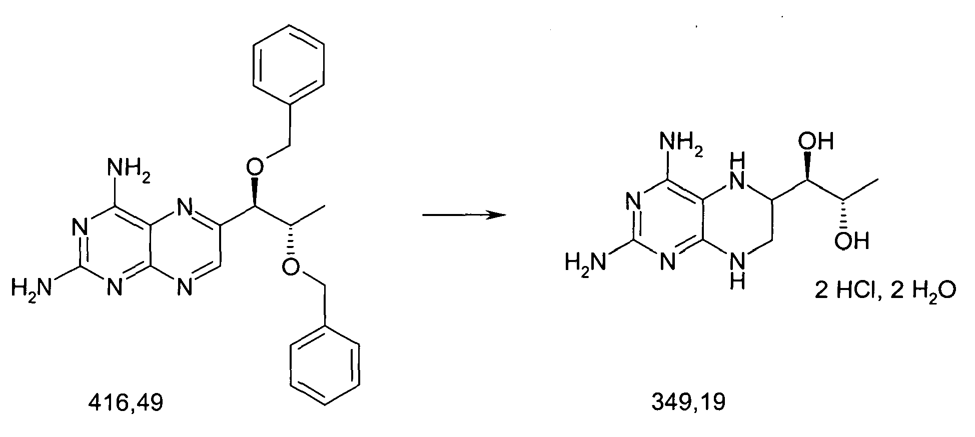

1,8 g of a platinum oxide catalyst were saturated with hydrogen at normal pressure in 50 mL 0,5N HCl in ethanol. After complete hydrogenation a solution of 1,8 g (1R,2S)-1,2-O-dibenzyl-1-(2,4-diamino-6-pteridinyl)-1,2-propanediol in 34 mL of 0,5N HCl in ethanol, was added and the reaction mixture was hydrogenated at normal pressure until 410 ml hydrogen were taken up. The catalyst was filtered from the solution via Celite-bed, the filter residue was washed three times with 20 mL ethanol each and the filtrate was concentrated to a volume of 80 mL under reduced pressure (40 °C bath temperature). Diethylether, previously flushed with nitrogen, was added, the reaction mixture was stirred for one hour at room temperature and subsequently left in a refrigerator for one additional hour. The precipitate was filtered under nitrogen atmosphere and dried in high vacuum for an hour. 761 mg Yellowish crystals were obtained. The mother liquor was evaporated to a volume of 40 ml. Again diethyl ether was added and the procedure given above was repeated. Additional 395 mg crystals were obtained.

### Purification:

651 mg of the hydrogenation product were dissolved in 6,5 mL methanol and treated with stirring with 45 mL dichloromethane. The resulting precipitate was stirred at room temperature for one hour and then filtered under nitrogen. The filter residue was washed with dichloromethane and sucked dry. The resulting crystals were dissolved in 12,5 mL 0,5N HCl in ethanol and diethyl ether was added slowly to the solution. Stirring was maintained for 1,5 hours, then the precipitate was filtered under nitrogen, washed with 10 mL diethyl ether. After drying in high vacuum 425 mg white (yellowish) crystals were obtained.

¹H-NMR (MeOD): δ = 3,91 - 3,48 (m, 5 H), 1,31 - 1,28 (dd, 3 H); ¹³C-NMR (MeOD): δ = 154,88, 154,84, 154,48, 154,36, 152,81, 152,61, 72,85, 72,67, 69,90, 69,65, 56,38, 56,04, 41,09, 38,08, 20,97, 20,33.

## Claims

1. A compound of the general formula VIII 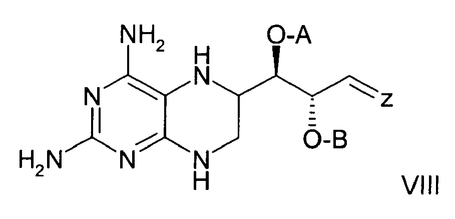 wherein A and B are independently from each other -H or protective groups for hydroxyl groups and =Z is a dithioacetal moiety or =Z means ― H, -H, with the proviso that the compound of the formula III 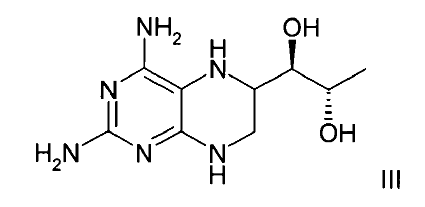 is excluded.

2. A compound of the general formula II, 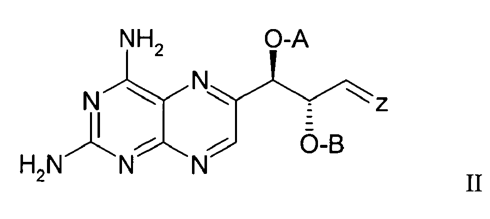 wherein A, B and =Z have the meaning as defined in claim 1.

3. A compound of the general formula I 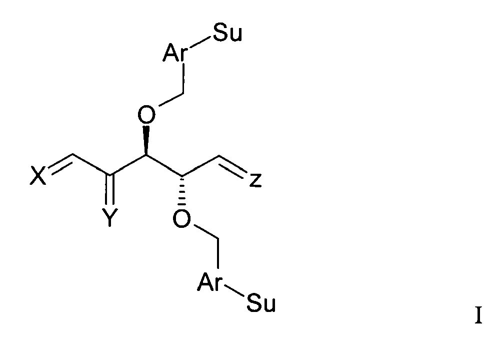 wherein the protective groups A and B as defined in claim 1 are independently from each other -CH₂-Ar-Su, and Ar has the meaning of a benzene ring, Su is from one to five substituents at the benzene ring and stands for branched or unbranched alkyl, branched or unbranched alkoxy, substituted or unsubstituted amino, halogen, nitro, cyano or trifluoromethyl and Z has the meaning as defined in claim 1, and =X has the meaning of =O, =NOR₁, =NNHR₁, with R₁ meaning H, unbranched or branched alkyl, unsubstituted or substituted phenyl, wherein =Y is =O or has the same meaning as =X and has in addition the meaning of -H, -OH, wherein =X may be either equal or unequal to =Y.

4. A process for the manufacture of a compound of the general formula I as defined in claim 3, comprising steps (i) to (vi):
(i) transformation of D-ribose into a dithioacetal of the general formula IV, 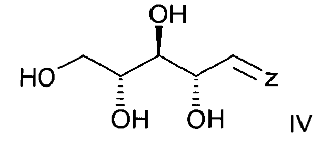 wherein =Z has the meaning of said thioacetal moiety as defined in claim 3,
(ii) ketal formation of compound IV to yield a compound of general formula V, 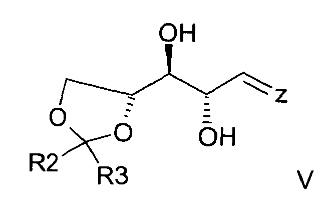 wherein R₂ and R₃ are independently from each other branched or unbranched alkyl, substituted or unsubstituted aryl or H,
(iii) benzyl ether type protection of the OH-groups of compound V to yield a compound of the general formula VI, 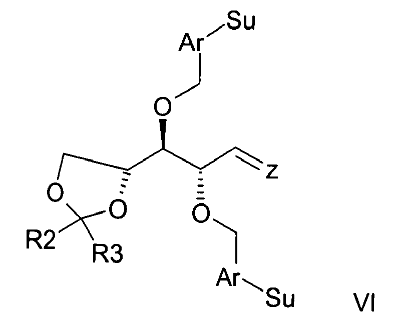 wherein -CH₂-Ar-Su have the same meaning as defined in claim 3, and wherein =Z has the meaning of said dithioacetal moiety or stands for -H, -H, in the latter meaning assigning a reaction step of a reductive cleavage of said thioacetal group prior to the introduction of -CH₂-Ar-Su,
(iv) cleavage of the ketal group in compound VI to yield protected sugar derivatives of the general formula VII 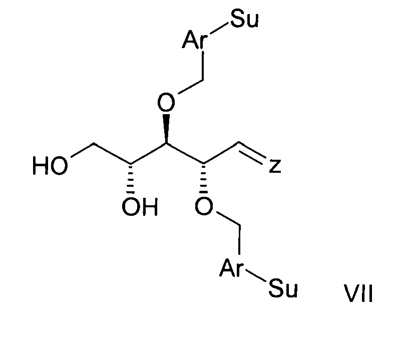
(v) subsequent step of an oxidation reaction to yield a compound of the general formula I, wherein =X and =Y are O,
(vi) and optionally modification of the compound obtained in step (v) to form a monoxime, dioxime or hydrazone to yield a compound of the general formula I, wherein =X has the meaning =NOR₁, =NNHR₁, with R₁ meaning H, unbranched or branched alkyl, unsubstituted or substituted phenyl, in which =Y is =O or has the same meaning as =X.

5. A process for the manufacture of a compound of the general formula II as defined in claim 2, wherein A and B have the meaning of -CH₂-Ar-Su as defined in claim 3, comprising step (i):
(i) reaction of tetraaminopyrimidine with a compound of the general formula I as defined in claim 3 or with a compound manufactured according to a process as defined in claim 4.

6. A process for the manufacture of a compound of the general formula VIII as defined in claim 1, wherein A and B have the meaning of-CH₂-Ar-Su as defined in claim 3, comprising steps (i) and (ii):
(i) reaction of tetraaminopyrimidine with a compound of the general formula I as defined in claim 3 or with a compound manufactured according to a process as defined in claim 4,
(ii) hydrogenation of the compound obtained in step (i).

7. A process for the manufacture of the compound of the formula III 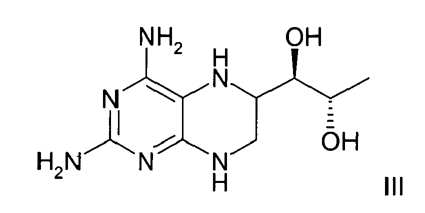 comprising a process as claimed in any one of claims 4 to 6.

8. A process for the manufacture of the compound of the formula III comprising a process as claimed in claim 6, wherein step (ii) is carried out by a catalytic hydrogenation method under acidic conditions.

9. A process for the manufacture of the compound of the formula III comprising a process as claimed in claim 6, further comprising step (iii):
(iii) cleavage of the protective groups -CH₂-Ar-Su of the compound obtained in step (ii) by oxidation, acid hydrolysis or irradiation.

10. A use of D-ribose for the manufacture of a compound of the general formula I as defined in claim 3 or for a compound of the general formula II as defined in claim 2 or for a compound of the general formula VIII as defined in claim 1 or for a compound of the formula III or a use of D-ribose for the manufacture of a compound manufactured according to a process as claimed in any one of claims 4 to 9.

11. A process as claimed in any one of claims 4 to 9 comprising the use of D-ribose.
